# EUROPEAN PATENT SPECIFICATION

(11) **EP 2 247 753 B1**
(45) Date of publication and mention of the grant of the patent: **25.06.2014**
(21) Application number: 09709391.8
(22) Date of filing: 29.01.2009
(51) Int. Cl.: C12Q 1/68

(54) **URINE TRANSPORT MEDIUM**
URINTRANSPORTMITTEL
MILIEU POUR LE TRANSPORT D URINE

(30) Priority: 01.02.2008 US 25393
(43) Date of publication of application: 10.11.2010
(73) Proprietor: Siemens Healthcare Diagnostics Inc., Tarrytown, NY 10591 (US)
(72) Inventor: SHERMAN, David, Davis CA 95618 (US); MENG, Qi, Fremont CA 94539 (US)
(74) Representative: Maier, Daniel Oliver
(86) International application number: PCT/US2009/032324
(87) International publication number: WO 2009/099854

(56) References cited:
- WO-A1-96/41810
- US-A- 4 378 429
- US-A- 5 482 834
- US-A- 5 637 687
- US-A- 5 945 525
- US-A- 5 945 525
- US-A1- 2006 094 015
- US-B1- 6 458 546
- US-B2- 6 537 745
- US-B2- 6 537 745
- US-B2- 6 618 679
- GOMES J P ET AL: "Correlating Chlamydia trachomatis infectious load with urogenital ecological success and disease pathogenesis", MICROBES AND INFECTION, ELSEVIER, PARIS, FR, vol. 8, no. 1, 1 January 2006 (2006-01-01) , pages 16-26, XP025243372, ISSN: 1286-4579, DOI: DOI:10.1016/J.MICINF.2005.05.014 [retrieved on 2006-01-01]
- ANONYMOUS: "High Pure PCR Template Preparation Kit", INTERNET CITATION, April 2005 (2005-04), XP002439230, Retrieved from the Internet: URL:http://web.archive.org/web/20051025172 652/http://www.roche-aplied-sci ence.com/pack-insert/1796828a.pdf [retrieved on 2007-06-25]

## Description

### FIELD OF THE INVENTION

The invention is related to methods for stabilizing patient samples for analysis. Specifically, the invention is related to the stabilization of patient samples such as urine for subsequent analysis of pathogens.

### BACKGROUND OF THE INVENTION

Early detection is an essential component of public health programs to control sexually transmitted disease. The goals of early detection and early treatment include interruption of the chain of transmission, prevention of long-term sequelae, and reduction of duration of infectiousness to limit the risk of co-infection. Early detection may also prevent over-treatment, which is a major concern due to widespread antibiotic resistance of certain pathogens.

Isolation of pathogens such as *Chlamydia trachomatis* and *Neisseria gonorrhoeae* and in cell culture has been the traditional method for laboratory diagnosis and has remained the method of choice for medico-legal specimens because of its specificity. However, this method requires stringent transport conditions to preserve specimen viability and has a turnaround time of 2 to 3 days. In many settings, cell culture has been replaced by more rapid tests based on antigen detection by direct fluorescent antibody staining, enzyme immunoassays, and enzyme-linked immunosorbent assays (ELISA), which have less demanding transport requirements and can provide results on the same day. However, these methods are still laborious and time-consuming and, more importantly, lack sensitivity as screening assays, especially for asymptomatic patients.

More recently, nucleic acid-based hybridization probe tests have been developed for direct detection of pathogens that cause sexually transmitted disease, such as *Chlamydia trachomatis* and *Neisseria gonorrhoeae.* These tests offer higher specificity but no substantial improvement on sensitivity. Furthermore, most of these tests are performed on endocervical or urethral specimens, which are obtained using invasive sampling procedures. Nucleic acid amplification assays based on polymerase chain reaction (PCR), ligase chain reaction (LCR), strand-displacement amplification (SDA), or transcription-mediated amplification (TMA) technology are now available. In addition to offering all the advantages of non-culture tests in terms of ambient specimen transport, batching automation, and rapid processing time, these assays provide higher specificity and a sensitivity approaching 100%. Furthermore, they can be performed on less invasive clinical specimens such as urine. All these advantages make nucleic acid amplification assays particularly suited for detection of asymptomatic infection and as a screening tool.

The practice of existing nucleic acid amplification assays for detection of pathogens that cause sexually transmitted disease still exhibits certain disadvantages and limitations, however. Clinical samples collected for analysis of the possible presence and quantity of pathogens are often subjected to lysis of any microbial cells present followed by extraction and analysis of nucleic acids. Often such samples cannot be processed immediately after they are obtained, requiring measures to stabilize the target nucleic acids in the sample.

Typically, a patient sample such as a urine or blood sample is first obtained in a physician's office, a clinic, a hospital, or even in a patient's home or at a remote location. Subsequently, the sample is transported to a central facility for high throughput analysis or screening. Unless special measures are taken to preserve the sample, such as refrigeration, freezing, or chemical preservation, then hours, days, or weeks may transpire between collection of the sample and its analysis. Liquid specimens are typically refrigerated or frozen, requiring costly equipment and carefully managed handling procedures to ensure accurate results. Use of dried specimens can result in loss of target nucleic acids and extraction difficulties, again yielding less reliable results.

Highly sensitive detection methods such as PCR can detect even minor degradation or loss of nucleic acids in a collected patient sample, potentially leading to false negatives unless appropriate stabilization measures are taken. Yet, biological specimens typically contain enzymes such as nucleases that will rapidly break down target nucleic acids if contacted at a temperature sufficiently high to support catalysis. Furthermore, cells of a pathogen, such as bacterial cells, are apt to further grow and multiply in biological material which is not frozen immediately after collection. Therefore, methods and compositions are needed for stabilizing nucleic acids in a sample such as a urine sample from a patient for storage and transport without freezing or refrigeration until analysis is possible. Such methods and compositions should preferably avoid interference with methods for purifiying and analyzing target nucleic acids.

### BRIEF SUMMARY OF THE INVENTION

The invention provides methods for the stabilization of patient samples containing nucleic acids for subsequent analysis. The invention is particularly useful for stabilizing urine samples used to screen for the presence of infectious agents such as *Chlamydia trachomatis* and *Neisseria gonorrhoea.* By adding a stabilizer solution according to the invention, target nucleic acids in a sample become stable enough to allow reproducible analysis by quantitative PCR after several weeks of storage at room temperature.

One aspect of the invention is a method of stabilizing a sample from a subject for nucleic acid analysis. The method includes adding an aliquot of the sample to a stabilizer solution consisting of a chaotrope, a detergent, and a buffer to form a stabilized sample.

Another aspect of the invention is a method of screening for the presence of a pathogen in a urine sample by nucleic acid analysis. The method includes adding a urine transport medium to an aliquot of the urine sample to form a stabilized urine sample. The urine transport medium consists a chaotrope, a non-ionic detergent, and a buffer. The stabilized sample is stored at 15-30°C for up to 28 days. Following storage, magnetic particles are added to the stabilized sample in order to bind nucleic acids from the stabilized sample. Once bound to the magnetic particles, the bound nucleic acids are isolated using a magnet. Finally, one or more target nucleic acids are amplified from the isolated nucleic acids using one or more primer sets specific for the pathogen. In certain embodimentd, quantitative PCR is performed using the isolated nucleic acids and the target-specific primers, and the presence or absence of the pathogen in the sample is indicated by the Ct value (threshold cycle) from the reaction.

### BRIEF DESCRIPTION OF THE DRAWINGS

Figure 1 shows a plot of the percent detection of a *Neisseria gonorrhoeae* specific target sequence vs. the target sequence concentration in a stabilized urine sample.
Figure 2 shows the average threshold cycle (Ct) for detection of a *Neisseria gonorrhoeae* specific target sequence as a function of storage time under the conditions indicated.

### DETAILED DESCRIPTION OF THE INVENTION

The inventors have discovered that a biological sample intended for quantitative nucleic acid analysis unexpectedly can be stabilized for several weeks at room temperature by adding to the sample a chaotrope, a detergent, and a pH buffering agent. A stabilizer solution consisting of these three chemical agents maintains the overall detection rate of the assay out to at least one month of storage at room temperature and even immediately lowers the limit of detection for a nucleic acid target. The invention provides stabilizer solution compositions, methods for stabilizing a biological sample for nucleic acid analysis, methods for screening for the presence or absence of one or more pathogens in a patient sample, methods for quantifying one or more pathogens in a patient sample, and kits for use with the compositions and methods. In particular, the invention provides a urine transport medium that permits improved stability and ease of transportation of urine samples to be used in large scale screening and diagnosis for the widespread agents of sexually transmitted disease, *Chlamydia trachomatis* and *Neisseria gonorrhoeae,* without sacrificing sensitivity, accuracy, or reliability.

The term "sample", "test sample", or "biological sample" as used herein, refers to any liquid or solid material suspected of containing nucleic acids of a desired target, especially of a pathogen. A test sample may be, or may be derived from, any biological tissue or fluid that can contain target nucleic acids. Frequently, the sample will be a "clinical sample", i.e., a sample obtained or isolated from a patient to be tested for infection. Such samples include, but are not limited to, bodily fluids which contain cellular materials and may or may not contain cells, e.g., blood, plasma, serum, urine, seminal fluid, saliva, ocular lens fluid, lymphatic fluid, amniotic fluid, and the like; endocervical, urethral, rectal, vaginal, vulva-vaginal, nasopharyngeal and pulmonary samples; and archival samples with known diagnosis. Test samples may also be sections of tissues such as frozen sections. The term "sample" also encompasses any material derived by processing a biological sample. Derived materials include, but are not limited to, cells (or their progeny) isolated from the sample, cell components, and nucleic acid molecules extracted from the sample. Processing of biological samples to obtain a test sample may involve one or more of: filtration, distillation, centrifugation, extraction, concentration, dilution, purification, inactivation of interfering components, addition of reagents, and the like.

The terms "individual, "subject' and "patient' are used herein interchangeably. They refer to a human being that can be the host of infection by a pathogen such as a bacterium, but may or may not be infected by the bacterium. The terms do not denote a particular age, and thus encompass adults, children, newborns, as well as fetuses.

The terms "nucleic acid", "nucleic acid molecule" and "polynucleotide" are used herein interchangeably. They refer to a deoxyribonucleotide or ribonucleotide polymer in either single-stranded or double-stranded form, and unless otherwise stated, encompass known analogs of natural nucleotides that can function in a similar manner as naturally occurring nucleotides. The terms encompass nucleic acid-like structures with synthetic backbones, as well as amplification products.

As used herein, the term "amplification" refers to a method or process that increases the representation of a population of specific nucleic acid sequences in a sample. Amplification methods (such as polymerase chain reaction or PCR) are known in the art and are discussed in more detail below.

The present invention can be employed in conjunction with any of a variety of nucleic acid amplification methods well-known in the art (see, for example, A.R. Kimmel and S.L. Berger, Methods Enzymol. 1987, 152: 307-316; J. Sambrook et al, "Molecular Cloning: A Laboratory Manual", 1989, 2nd Ed., Cold Spring Harbour Laboratory Press: New York, NY; "Short Protocols in Molecular Biology", F.M. Ausubel (Ed.), 2002, 5th Ed., John Wiley & Sons: Secaucus, NJ). Such nucleic acid amplification methods include, but are not limited to the Polymerase Chain Reaction (or PCR, described in, for example, "PCR Protocols: A Guide to Methods and Applications", M.A. Innis (Ed.), 1990, Academic Press: New York; "PCR Strategies", M.A. Innis (Ed.), 1995, Academic Press: New York; "Polymerase chain reaction: basic principles and automation in PCR: A Practical Approach", McPherson et al. (Eds.), 1991, IRL Press: Oxford; Saiki et al, Nature, 1986, 324: 163; and U.S. Pat. Nos. 4,683,195, 4,683,202 and 4,889,818 ); and variations thereof including TaqMan-based assays (Holland et al, Proc. Natl. Acad. Sci., 1991, 88: 7276-7280), and reverse transcriptase polymerase chain reaction (or RT-PCR, described in, for example, U.S. Pat. Nos. 5,322,770 and 5,310,652).

The terms "target sequence" and "target nucleic acid" are used herein interchangeably. They refer to a nucleic acid sequence, the presence or absence of which is desired to be detected. In the context of the present invention, a target sequence preferably includes a nucleic acid sequence to which oligonucleotide primers will complex. The target sequence may also include a probe-hybridizing region with which a probe will form a stable hybrid under desired conditions. A target sequence may be single-stranded or double-stranded.

The terms "primer" and "amplification primer" axe used herein interchangeably they refer to an oligonucleotide which is capable of acting as a point of initiation of synthesis of a primer extension product that is a complementary strand of DNA, when placed under suitable conditions (e.g., buffer, salt, temperature and pH) in the presence of nucleotides and an agent for nucleic acid polymerization (e.g., a DNA- dependent or RNA-dependent polymerase). The primer is preferably single-stranded for maximum efficiency in amplification, but may alternatively be double-stranded. If double-stranded, the primer may first be treated (e.g., denatured) to allow separation of its strands before being used to prepare extension products. Such a denaturation step is typically performed using heat, but may alternatively be carried out using alkali, followed by neutralization. A typical primer contains about 10 to about 35 nucleotides in length of a sequence substantially complementary to the target sequence. However, a primer can also contain additional sequences. For example, amplification primers used in Strand Displacement Amplification (SDA) preferably include a restriction endonuclease recognition at site 5' to the target binding sequence (see, for example, U.S. Pat. Nos. 5,270,184 and 5,455,166). Nucleic Acid Sequence Based Amplification (NASBA), and Transcription-Mediated Amplification (TMA) primers preferably include an RNA polymerase promoter linked to the target binding sequence of the primer. Methods for linking such specialized sequences to a binding target sequence for use in a selected amplification reaction are well-known in the art.

As used herein, the term "primer set" refers to two or more primers which together are capable of priming the amplification of a nucleotide sequence of interest (e.g., a target sequence within the OFR1 gene, the dcmG gene or the pivNG gene of *Neisseria gomorrhea*). In certain embodiments, the term "primer set" refers to a pair of primers including a 5' (upstream) primer (or forward primer) that hybridizes with the 5'- end of the nucleic acid sequence to be amplified and a 3' (downstream) primer (or reverse primer) that hybridizes with the complement of the sequence to be amplified. Such primer sets or primer pairs are particularly useful in PCR amplification reactions.

The term "amplification conditions", as used herein, refers to conditions that promote annealing and/or extension of primer sequences. Such conditions are well- known in the art and depend on the amplification method selected. Thus, for example, in a PCR reaction, amplification conditions generally comprise thermal cycling, i.e., cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, pH, and the like.

As used herein, the term "amplification reaction reagents", refers to reagents used in nucleic acid amplification reactions and may include, but are not limited to, buffers, reagents, enzymes having reverse transcriptase and/or polymerase activity or exonuclease activity; enzyme cofactors such as magnesium or manganese; salts; and deoxynucleotide triphosphates (dNTPs) such as deoxyadenosine triphosphate (dATP), deoxyguanosine triphosphate (dGTP), deoxycytidine triphosphate (dCTP), deoxythymidine triphosphate (dTTP) and deoxyuridine triphosphate (dUTP).

The terms "probe" and "detection probe" are used herein interchangeably and refer to an oligonucleotide capable of selectively hybridizing to at least a portion of a target sequence under appropriate conditions (e.g., a portion of a target sequence that has been amplified). In general, a probe sequence is identified as being either "complementary" {i.e., complementary to the coding or sense strand (+)), or "reverse complementary" (i.e., complementary to the anti-sense strand (-)). In certain embodiments, a detection probe is labeled with a detectable moiety.

The terms "labeled" and "labeled with a detectable agent (or moiety)" are used herein interchangeably to specify that an entity (e.g., an oligonucleotide detection probe) can be visualized, for example following binding to another entity (e.g., an amplification reaction product or amplicon). Preferably, the detectable agent or moiety is selected such that it generates a signal which can be measured and whose intensity is related to (e.g., proportional to) the amount of bound entity. A wide variety of systems for labeling and/or detecting nucleic acid molecules are well-known in the art. Labeled nucleic acids can be prepared by incorporation of, or conjugation to, a label that is directly or indirectly detectable by spectroscopic, photochemical, biochemical, immunochemical, electrical, optical, chemical or other means. Suitable detectable agents include, but are not limited to, radionuclides, fluorophores, chemiluminescent agents, microparticles, enzymes, colorimetric labels, magnetic labels, haptens, Molecular Beacons, and aptamer beacons.

The use of physically linked fluorescent reporter/quencher molecule pairs is also within the scope of the invention. The use of such systems in TaqMan assays (as described, for example, in U.S. Pat. Nos. 5,210,015; 5,804,375; 5487,792 and 6214,979) or as Molecular Beacons (as described, for example in, S. Tyagi and F.R. Kramer, Nature Biotechnol. 1996, 14: 303-308; S. Tyagi et al, Nature Biotechnol. 1998, 16: 49-53; L.G. Kostrikis et ah, Science, 1998, 279: 1228-1229; D.L. Sokol et ah, Proc. Natl. Acad. Sci. USA 1998, 95: 11538-1 1543; S.A. Marras et ah, Genet. Anal. 1999, 14: 151-156; and U.S. Pat. Nos. 5,846,726, 5,925,517, 6,277,581 and 6,235,504) is well-known in the art. With the TaqMan assay format, products of the amplification reaction can be detected as they are formed in a so-called "real-time" manner. As a result, amplification product/probe hybrids are formed and detected while the reaction mixture is under amplification conditions.

In some embodiments of the present invention, the PCR detection probes are TaqMan-like probes that are labeled at the 5'-end with a fluorescent moiety and at the 3'-end with a quencher moiety. Suitable fluorophores and quenchers for use with TaqMan-like probes are disclosed in U.S. Pat. Nos. 5,210,015; 5,804,375; 5,487,792; and 6,214,979; and WO 01/86001. Examples of quenchers include, but are not limited to DABCYL (Le., 4-(4r-dimethylaminophenylazo)-benzoic acid) succinimidyl ester, diarylrhodamine carboxylic acid, succinimidyl ester (or QSY-7), and 4',5'-dinitrofluorescein carboxylic acid, succinimidyl ester (or QSY-33) (all available, for example, from Molecular Probes), quencher 1 (Q1; available from Epoch Biosciences, Bothell, WA), or "Black hole quenchers" BHQ-I, BHQ-2, and BHQ-3 (available from BioSearch Technologies, Inc., Novato, CA). In certain embodiments, the PCR detection probes are TaqMan-like probes that are labeled at the 5' end with FAM and at the 3' end with a Black Hole Quencher.

A "tail" of normal or modified nucleotides can also be added to oligonucleotide probes for detectability purposes. A second hybridization with nucleic acid complementary to the tail and containing one or more detectable labels (such as, for example, fluorophores, enzymes or bases that have been radioactively labeled) allows visualization of the amplicon/probe hybrids (see, for example, the system commercially available from Enzo Biochem. Inc., New York, NY). Another example of an assay with which the inventive oligonucleotides are useful is a signal amplification method such as that described in U.S. Pat. No. 5,124,246. In that method, the signal is amplified through the use of amplification multimers, polynucleotides which are constructed so as to contain a first segment that hybridizes specifically to the "tail" added to the oligonucleotide probes, and a multiplicity of identical second segments that hybridize specifically to a labeled probe. The degree of amplification is theoretically proportional to the number of iterations of the second segment. The multimers may be either linear or branched. Branched multimers may be in the shape of a fork or a comb.

A sample is "stabilized" according to the invention when the target nucleic acids in the sample have been protected from degradation due to forces originating within the sample, such as nucleases, and forces from outside the sample, such as storage for extended periods of days to weeks at room temperature. Stabilization of a sample according to the invention lasts for at least one month, at least two months, or longer. During the period of stabilization, a target nucleic acid remains detectable at essentially the same limit of detection as at the outset of the stabilization period, i.e., just after the addition of stabilizer solution or components of a stabilizer solution. A "stabilizer solution" as used herein is a solution containing chemical components which, according to the invention, will render a sample stabilized when added in an appropriate amount to the sample. A stabilizer solution can be added to a sample either as a liquid or as solid components that dissolve in the sample to produce the appropriate final concentrations of all ingredients, the same as if a liquid stabilizer solution had been added to the sample. A "urine transport medium" as used herein is a stabilizer solution for use with a urine sample, and which when added in appropriate amount to a urine sample will stabilize target nucleic acids in the urine sample. A "stabilized sample" as used herein is a sample that has been stabilized by the addition of stabilizer solution or through the addition of chemical components as solids or concentrated stock solutions directly into an aliquot of the sample.

The "limit of detection" or "LoD" as used herein refers to the concentration of a target nucleic acid sequence that can be detected with 95% confidence using a conventional nucleic acid detection or quantification method, such as quantitative PCR. The LoD can be expressed, for example, as the number of copies of the target sequence contained in a specific volume or aliquot of a sample. Unless otherwise specified, the LoD of a target refers to the LoD determined under optimal storage and assay conditions, i.e., assayed immediately after obtaining or preparing the sample, avoiding any degradation of the target such as might occur during storage or transportation.

### Stabilizer Solution

A stabilizer solution, or a stabilized sample, in accordance with the invention consists of three chemical agents: a chaotropic agent, a detergent, and a buffer. Without intending to limit the invention to any particular mechanism, a stabilizer solution lyses cells such as bacterial cells in a sample and inactivates enzymes such as nucleases by at least partially denaturing proteins. It does this preferably without leading to precipitation, cross-linking, aggregation, gelation, hydrolysis, or other forms of chemical or physical alteration of sample components. It further has minimal or no impact on either subsequent binding of nucleic acids to silica magnetic particles or on nucleic acid amplification reactions such as quantitative PCR.

Any chaotrope is suitable for use in a stabilizer solution according to the invention if used at sufficiently high concentration to contribute to stabilizing a target nucleic acid against degradation over time when stored at room temperature. A chaotropic agent is a chemical agent that disrupts the three dimensional structure of macromolecules, especially proteins and nucleic acids. Chaotropic agents interfere with non-covalent intra-molecular interactions such as hydrogen bonds, Van der Waals forces, and hydrophobic bonds. Examples of suitable chaotropes include guanidinium salts such as guanidine thiocyanate, guanidine isothiocyanate, and guanidine hydrochloride; urea; and lithium or potassium salts of bromide, iodide, or perchlorate. Chaotropes are generally used at high concentrations that shield charged groups in macromolecules, thereby weakening ionic interactions, or increase the dipole moment of the solvent, thereby weakening hydrogen bonds. The final concentration of the chaotrope component of a stabilized sample in can be from about 0.1 to about 3 M, or about 0.5 to about 1.5 M, or about 0.2 to about 1.1 M, and preferably is about 1M. The upper limit of the chaotrope concentration is in principle only limited by the solubility of the chaotropic salt in the biological sample and in the stabilizer solution. Consequently, the concentration of chaotrope in a stabilizer solution according to the invention will depend on the dilution factor when the stabilizer solution is added to a sample or an aliquot of a sample. For example, if the chaotrope is 5M in a stabilizer solution, and 400 µL of stabilizer solution are added to a 2 mL aliquot of a urine sample, then the final concentration of the chaotrope in the stabilized sample will be 0.83 M. The concentration of chaotrope in a stabilizer solution is typically about 0.5 - 8 M, and in some cases up to 10 M. In different embodiments, the chaotrope concentration in a stabilizer solution can be about 1 - 6 M, or about 1 - 5.5 M. Prefereably, the chaotrope concentration in a stabilizer solution is about 5 M.

The use of a detergent in a stabilizer solution according to the invention insures that bacterial or other target cells are lysed and release target nucleic acids into the stabilized sample, where they are accessible to nucleic acid binding agents. A sufficiently high concentration of detergent contributes to denaturation of proteins and other macromolecules in the sample and also helps prevent aggregation of proteins and other components over the storage period. The detergent component of a stabilized sample contributes to stabilizing target nucleic acids against degradation over time when stored at room temperature. Any detergent can be used. Preferably, the detergent does not gel or precipitate, or cause aggregation or precipitation of protein or nucleic acid components, during storage of a stabilized sample at room temperature. Examples of suitable non-ionic detergents include polyoxyethylene esters of alkylphenols (e.g., Triton-X-100 (polyoxyethylene octyl phenyl ether), Triton-X-114), triblock copolymers of ethylene oxide and propylene oxide (e.g., Pluronic), Tween-20, Tween-80, Brij-35, octyl-β-D-glucosylpyranoside, n-dodecyl-β-D-maltoside, and derivatives or analogs thereof. Ionic detergents (e.g., sodium dodecyl sulfate) or zwitterionic detergents also can be used. The suitable concentration range will depend on the detergent; however, nonionic detergents generally can be used at a final concentration in the stabilized sample in different embodiments ranging from about 0.5 - 5 % v/v, or about 1 - 5 % v/v, or about 0.4 - 2% v/v, or preferably about 1.5 - 3 v/v %. The concentration of detergent in a stabilizer solution is typically about 2 - 10 %. In different embodiments, the detergent concentration in a stabilizer solution can be about 1 - 10 % v/v, or about 1 - 5 % v/v, or about 2 - 5 % v/v. Preferably, the detergent concentration in a stabilizer solution is about 10 % v/v for a nonionic detergent.

A pH buffer is added to a stabilizer solution according to the invention to improve stabilization of target nucleic acids during storage. Any buffer can be used that provides a pH in the range of pH 3.5 - 9.0. Preferably the buffer is adjusted to a pH in the range of 4.0 - 8.0. A pH of 4.1 is preferred. Any suitable buffering agent can be used. Sodium acetate is preferred. The concentration of the buffering agent in a stabilized sample is preferably at a concentration in the range of about 5 mM to about 200 mM, or about 10 mM to about 100 mM, or preferably at about 15-25 mM. Depending on the dilution factor when added to an aliquot of sample, a stabilizer solution according to the invention has about 20 mM to about 1 M of a buffering agent, preferably about 100 mM.

### Preparing a Stabilized Sample

According to methods of the present invention, the presence of a target nucleic acid sequence in a sample can be determined by amplifying the target nucleic acid using target-speicific primers. The sample can be any liquid or solid biological material suspected of comprising such target sequences. In certain embodiments, preferred test samples include urine (e.g., first catch urine), seminal fluid, saliva, ocular lens fluid, lymphatic fluid, endocervical, urethral, rectal, vaginal, vulva-vaginal, and nasopharyngeal samples. Other preferred test samples include PAPS-smear specimens.

Test samples will often be obtained or isolated from patients suspected of being infected with a pathogen such as *Neisseria gonorrhoeae* or *Chlamydia trachomatis.* A test sample may be used for stabilization and analysis according to the invention without further treatment after isolation. Alternatively, the test sample may be processed before stabilization and analysis, for example, to release target nucleic acids from cells that contain them, or to first isolate cells from the sample. Methods of nucleic acid extraction are well-known in the art and include chemical methods, temperature methods, and mechanical methods (see, for example, J. Sambrook et al, "Molecular Cloning: A Laboratory Manual", 1989, 2nd Ed., Cold Spring Harbour Laboratory Press: New York, NY). There are also numerous different and versatile kits that can be used to extract nucleic acids from biological samples that are commercially available. Cells can be isolated by centrifugation or filtration. However, such pre-treatments generally are not necessary when using a stabilizer solution according to the invention, particularly if the sample is a liquid such as urine. A stabilizer solution of the invention is generally capable of lysing cells and also of denaturing enzymes such as nucleases that might otherwise lead to degradation of target nucleic acids.

A stabilized sample is prepared by adding an aliquot of stabilizer solution to an aliquot of the sample according to an appropriate dilution factor so as to achieve the desired final concentrations of stabilizer components (chaotrope, detergent, buffer). The dilution factor can be any amount consistent with solubility limits for preparing a concentrated stabilizer solution to be diluted into an aliquot of the sample. Generally, the ratio of added stabilizer solution volume to sample aliquot volume is in the range of about 0.02 to about 1, or about 0.04 to about 0.25. Preferably the ratio is about 0.2. Alternatively, solid or concentrated liquid chemical stabilizer components can be dissolved from stocks or from a lyophilized mixture into each sample aliquot for stabilization. Sample aliquots and stabilizer solution or chemicals can be combined by pipetting, pouring, or by dissolving components or a mixture of components into a liquid, as desired. Optionally, after combining a sample aliquot with stabilizer chemicals or stabilizer solution, the resulting stabilized sample can be mixed by hand, by using a vortex mixer, by stirring, by inverting a capped tube, or by another method as appropriate. Preferably, the sample aliquot and stabilizer solution are mixed in a sterile disposable tube or vial and provided with an airtight seal or cap.

Optionally, in addition to adding stabilizer solution or the components of stabilizer solution to the sample aliquot, one or more reagents for nucleic acid binding, isolation, purification, or amplification can additionally be added at the time of preparing the stabilized sample, either prior to storage or during the storage and/or transportation period prior to nucleic acid analysis. For example, silica coated magnetic particles can be added to a stabilized sample, so that target nucleic acids bind to the particles during the storage and/or transportation period.

### Methods of Analyzing Stabilized Samples for Presence of a Target Sequence

The invention further provides a method of screening for the presence of a pathogen in a urine sample by nucleic acid analysis. The method includes the steps of (a) adding a urine transport medium to an aliquot of the urine sample to form a stabilized urine sample containing a chaotrope, a non-ionic detergent, and a buffer; (b) storing the stabilized sample for a period of time at a temperature in the range from about 0°C to about 40°C (or from about 0°C to about 4°C, preferably at 15-30°C); (c) adding magnetic particles to the stabilized sample, wherein the particles bind nucleic acids from the stabilized sample; (d) isolating the bound nucleic acids from step (c) using a magnet; (e) amplifying one or more target nucleic acids from the isolated nucleic acids obtained in step (d) using one or more primer sets specific for the pathogen, wherein the step of amplifying includes performing quantitative polymerase chain reaction, and wherein the presence or absence of the pathogen in the sample is indicated by the Ct value from the reaction. In certain embodiments of the method, the stabilized urine sample contains from about 0.2 to about 1.1 M guanidine thiocyanate, about 0.4 to about 2 %v/v Triton-X-100, and from about 4 to about 20 mM sodium acetate at pH 4.1. In one embodiment of the method, the urine transport medium is a solution containing 5 M guanidine thiocyanate, 10 % v/v Triton-X-100, and 0.1 M sodium acetate at pH 4.1. The method can be used, for example, to screen for *Neisseria gonorrhoeae* or *Chlamydia trachomatis* in patients suspected of harboring an infection of the urinary tract or a sexually-transmitted disease. The method can be used for mass screening efforts to identify persons affected by such a disease or even asymptomatic carriers of such a disease. By appropriately choosing the primer and/or probe sets used for nucleic acid analysis, the method can be carried out so as to identify and/or quantify two or more pathogens simultaneously.

### Nucleic Acid Analysis

Amplification of target sequences and detection of amplified target nucleic acids according to methods of the present invention may be performed using any known amplification and detection methodologies. In certain embodiments, detection of a pathogen such as *Neisseria gonorrhoeae* or *Chlamydia trachomatis* in a test sample is performed using a TaqMan assay, and the formation of amplification products is monitored in a real time manner by fluorescence, e.g., using real time or quantitative PCR. In these embodiments, probes will be used that are labeled with a fluorescent reporter at the 5' end and a quencher moiety at the 3' end, as described herein. Optimization of amplification conditions and selection of amplification reaction reagents suitable for a TaqMan assay format are within the skill in the art.

In certain embodiments, an internal control or an internal standard is added to the biological sample (or to purified nucleic acids extracted from the biological sample) to serve as a control for extraction and/or target amplification. The internal control generally includes a sequence that differs from the target sequence(s) and is capable of amplification by the primers used to amplify the target nucleic acid(s), preferably with a similar amplification efficiency. The use of an internal control allows monitoring of the extraction process, amplification reaction, and detection, and control of the assay performance. The amplified control and amplified target are typically distinguished at the detection step by using different probes (e.g., labeled with different detectable agents) for the detection of the control and the target.

The presence of a target in a test sample may be confirmed by repeating an assay according to the present invention using a different aliquot of the same biological test sample or using a different test sample (e.g., an endocervical swab if the first sample analyzed was a urine sample, or a urine sample collected at a different time). Confirmatory tests can also be performed by targeting a different region of a target pathogen's chromosome using a different set of target-specific primers. Alternatively or additionally, the presence of a target in a test sample may be confirmed by performing a different assay (i.e., an assay based on a different amplification or detection methodology). For example, if the first analysis was performed using a TaqMan assay, a second analysis may be carried out using a transcription-mediated amplification (TMA) reaction. Alternatively or additionally, the presence of a pathogen in a test sample may be confirmed by a different assay (e.g., isolation from cell culture).

The present invention also contemplates methods for simultaneously detecting the presence of two or more targets indicative of the same organism or different organisms in a test sample using a combination of at least two primer sets or primer/probe sets. In certain preferred embodiments, target sequences for both *Neisseria gonorrhoeae* and *Chlamydia trachomatis* are tested simultaneously. In certain preferred embodiments, the primer/probe set specific for *Chlamydia trachomatis* is one described in WO 2007/056398 and the primer/probe set specific for *Neisseria gonorrhoeae* is one described in WO 2007/117642.

### Kits

Basic material and reagents required for the detection of any desired target nucleic acid, preferably diagnostic for a specific pathogen such as *Neisseria gonorrhoeae* and *Chlamydia trachomatis* according to the present invention may be assembled together in a kit. In certain embodiments, kits comprise a stabilizer solution according to the invention, one or more primer sets or primer/probe sets, and optionally, amplification reaction reagents. An alternative to providing a ready to use stabilizer solution is to provide all of the dry ingredients for preparing a stabilizer solution and a container, such that the user can reconstitute or prepare the stabilizer solution by adding purified water.

Each kit preferably comprises the reagents that are required for a particular assay procedure. Thus, a kit adapted for use with NASBA preferably contains primers with a RNA polymerase promoter linked to the target binding sequence, while a kit adapted for use with SDA preferably contains primers including a restriction endonuclease recognition site 5' to the target binding sequence. Similarly, when the kit is adapted for use in a 5' nuclease assay, such as the TaqMan assay, the detection probes preferably contain at least one fluorescent reporter moiety and at least one quencher moiety.

Suitable amplification reaction reagents for inclusion in a kit are, for example, one or more of: buffers, reagents, enzymes having reverse transcriptase and/or polymerase activity or exonuclease activity, enzyme cofactors such as magnesium or manganese; salts; deoxynucleotide triphosphates (dNTPs) such as deoxyadenosine triphosphate (dATP), deoxyguanosine triphosphate (dGTP), deoxycytidine triphosphate (dCTP), deoxythymidine triphosphate (dTTP) and deoxyuridine triphosphate (dUTP) suitable for carrying out the amplification reaction. For example, a kit, adapted for use with NASBA, may contain suitable amounts of reverse transcriptase, RNase H and T7 RNA polymerase. Depending on the procedure, kits may further comprise one or more of: wash buffers and/or reagents, hybridization buffers and/or reagents, labeling buffers and/or reagents, and detection means. The buffers and/or reagents are preferably optimized for the particular amplification/detection technique for which the kit is intended. Instructions or protocols for using the kit reagents and for performing different steps of the procedure may also be included in the kit. Furthermore, kits may be provided with an internal control or reference standard as a check on the amplification procedure and to prevent occurrence of false negative test results due to failures in the amplification procedure. An optimal control sequence is selected in such a way that it will not compete with the target nucleic acid sequence in the amplification reaction. Kits may also contain reagents for the isolation of nucleic acids from biological specimens prior to amplification and/or for the purification or separation of target cells before nucleic acid extraction. For example, kits can include magnetic particles, such as silica-coated paramagnetic particles, for use in nucleic acid isolation.

The reagents may be supplied in a solid (e.g., lyophilized) or liquid form. Kits may optionally comprise different containers (e.g., vial, ampoule, test tube, flask or bottle) for each individual buffer and/or reagent. Each component will generally be suitable as aliquoted in its respective container or provided in a concentrated form. Other containers suitable for conducting certain steps of the amplification/detection assay may also be provided. The individual containers are preferably maintained in close confinement for commercial sale.

Kits may also contain instructions for using the amplification reaction reagents and primer sets or primer/probe described herein. Instructions for using kits according to one or more methods of the invention may include instructions for processing, stabilizing, and storing the biological sample, extracting nucleic acid molecules, and/or performing the test; instructions for interpreting the results obtained as well as a notice in the form prescribed by a governmental agency (e.g., FDA) regulating the manufacture, use or sale of pharmaceuticals or biological products.

### Example 1

### Effect of Stabilizer Solution on Detection of Neisseria gonorrhoeae in Urine

A dilution series of *Neisseria gonorrhoeae* (GC) cells was prepared by adding appropriate amounts from a heat-inactivated GC cell stock to a GC-negative pooled urine sample obtained from a public health service. One GC cell was assumed to possess 1030 copies of a *Neisseria gonorrhoeae* specific target sequence (see WO 2007/117642). The GC-urine dilution series was stored either with or without addition of a stabilizer solution (5M guanidine thiocyanate, 10% Triton-X-100, 0.1M sodium acetate, pH 4.1) (400 µL stabilizer solution added to 2 mL of GC-urine), and analysis by quantitative PCR was performed as described in WO 2007/117642 on the same day, with storage at room temperature. Thermocycling conditions were 10 min at 50°C, 15 min at 95°C, followed by 40 cycles of 15 sec at 95°C and 60 sec at 62°C. At least 10 replicates were run for each GC dilution. For a sample yielding an average Ct (threshold cycle) value of 38 or higher, GC was considered not detected, while GC was considered detected for a sample giving an average Ct of less than 38. The results are shown in Table 1.

**Table 1 Effect of Stabilizer on Limit of Detection**

| Stabilizer Added | GC Target Concentration (copies/mL) | Percent Detection |
|---|---|---|
| No | 5000 | 100 |
| No | 2500 | 100 |
| No | 1250 | 100 |
| No | 625 | 100 |
| No | 312 | 88.88 |
| No | 156 | 77.77 |
| Yes | 360 | 100 |
| Yes | 240 | 100 |
| Yes | 180 | 100 |
| Yes | 90 | 75 |
| Yes | 45 | 70 |
| Yes | 22.5 | 20 |

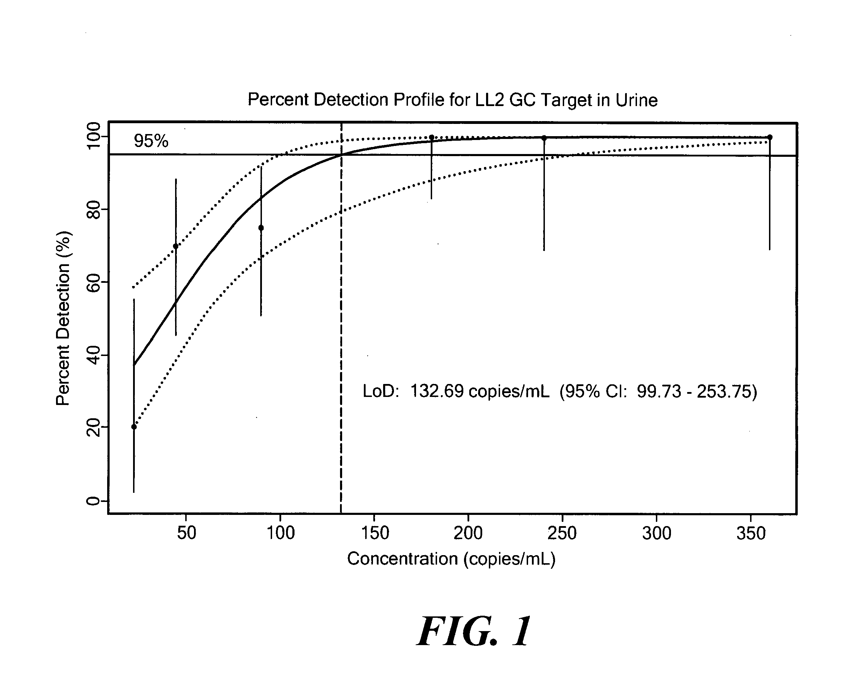

The data in Table 1 show that addition of stabilizer solution to the urine sample permitted detection down to significantly lower target concentrations. Figure 1 shows a plot of the percent detection of the *Neisseria gonorrhoeae* target sequence vs. the target sequence concentration for the stabilized urine sample. The limit of detection (LoD) was determined from logistic regression as the target sequence concentration corresponding to 95% detection. The LoD for stabilized GC-urine was 133 copies/mL, whereas the LoD for unstabilized GC-urine (not shown in Fig. 1) was 625 copies per mL.

### Example 2

### Effect of Stabilizer Concentration on Stabilization of Chlamydia trachomatis and Neisseria gonorrhoeae in Urine

The effectiveness of the stabilizer solution used in Example 1 was evaluated for addition to urine samples at various ratios of stabilizer to urine. Heat-inactivated *Neisseria gonorrhoeae* (GC) and *Chlamydia trachomatis* (CT) cells were added to a GC- and CT-negative pooled urine sample obtained from a public health service. The GC and CT cells were each added to three times their respective limits of detection. Various amounts of a stabilizer solution (5M guanidine thiocyanate, 10% Triton-X-100, 0.1M sodium acetate, pH 4.1) were added to the GC-urine and CT-urine samples to give the indicated final concentration of the stabilizer solution in the stabilized urine samples. Analysis was performed by quantitative PCR as described in Example 1. Primers and target sequences were as described in WO 2007/117642 and WO 2007/056398. The analysis was performed immediately after adding the stabilizer solution and repeated three days after adding the stabilizer solution and storage at 15-30°C. The results for the CT-urine samples are shown in Table 2, and for the GC-urine samples in Table 3. Ct%CV is the percent coefficient of variation of Ct. The results indicate that the full range of dilutions of stabilizing solution tested, from 4 to 20 % v/v, stabilized both GC and CT target sequences over at least three days at 15-30°C.

**Table 2 Stability of CT Target**

| **Timepoint** | **Stabilizer Concentration** | **Replicates** | **Ct Ave** | **Ct SD** | **Ct%CV** |
|---|---|---|---|---|---|
| Day 0 | 4% | 4 | 31.17 | 0.54 | 1.72 |
| | 6% | 4 | 31.81 | 0.6 | 1.88 |
| | 9% | 4 | 31.91 | 0.15 | 0.48 |
| | 17% | 4 | 32.54 | 0.55 | 1.69 |
| | 20% | 4 | 32.06 | 0.46 | 1.43 |
| Day 3 | 4% | 4 | 31.87 | 0.44 | 1.38 |
| | 6% | 4 | 31.31 | 0.54 | 1.73 |
| | 9% | 4 | 32.14 | 0.06 | 0.17 |
| | 17% | 4 | 32.1 | 0.17 | 0.53 |
| | 20% | 4 | 31.99 | 0.66 | 2.07 |

**Table 3 Stability of GC Target**

| **Timepoint** | **Stabilizer Concentration** | **Replicates** | **Ct Ave** | **Ct SD** | **Ct%CV** |
|---|---|---|---|---|---|
| Day 0 | 4% | 4 | 33.91 | 0.54 | 1.6 |
| | 6% | 3/4 | 34.15 | 0.65 | 1.9 |
| | 9% | 4 | 34.48 | 0.36 | 1.06 |
| | 17% | 4 | 34.95 | 1.01 | 2.89 |
| | 20% | 3/4 | 34.57 | 0.43 | 1.24 |
| Day 3 | 4% | 4 | 32.76 | 0.19 | 0.59 |
| | 6% | 4 | 31.86 | 0.56 | 1.77 |
| | 9% | 4 | 33.12 | 0.59 | 1.77 |
| | 17% | 4 | 33.84 | 0.14 | 0.42 |
| | 20% | 4 | 33.42 | 0.51 | 1.52 |

### Example 3

### Effect of Stabilizer Solution and Storage Conditions on Detection of Neisseria gonorrhoeae and Chlamydia trachomatis in Urine

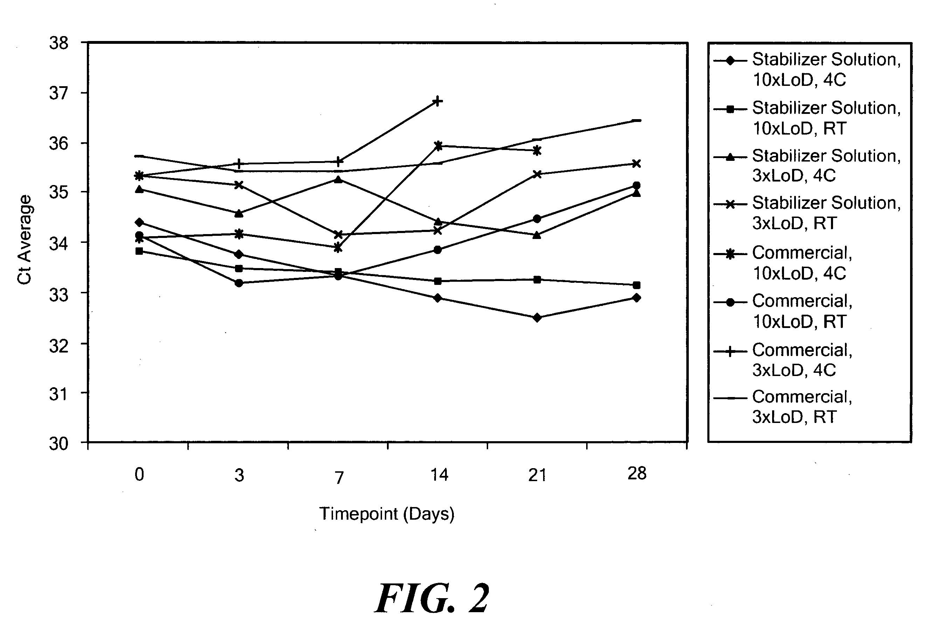

Stabilization of CT- and GC-urine samples with addition of either the stabilizer solution of Example 1 ("Stabilizer Solution") or a commercially available urine stabilizer ("Commercial") was investigated. In either case, 400 µL of stabilizer was added to 2 mL of urine. CT- and GC-urine were prepared as described in Example 2, except that dilutions were prepared at both 3x and 10x the LoD for each target. The samples were stored using the indicated time and temperature conditions prior to analysis by PCR as described in Example 1. The results are presented in Table 4, and are graphically depicted for the GC-urine samples in Fig. 2. The results show that the Stabilizer Solution of the present invention was more effective than the commercial stabilizer as measured by both Ct value and hit rate, particularly at longer periods of storage beyond 7 days.

**Table 4 Stability of GC Target at Various Storage Conditions**

| **Timepoint** | **Stabilizer** | **Storage** | **Target Conc** | **Average Ct** | **Standard Deviation** | **% CV** | **Hit Rate** |
|---|---|---|---|---|---|---|---|
| **Day 0** | Stabilizer Solution | 4C | 10x LoD | 34.39 | 0.89 | 2.59 | 8 out of 8 |
| | Stabilizer Solution | 15-30C | 10x LoD | 33.84 | 0.90 | 2.66 | 8 out of 8 |
| | Stabilizer Solution | 4C | 3x LoD | 35.05 | 0.28 | 0.79 | 4 out of 8 |
| | Stabilizer Solution | 15-30C | 3x LoD | 35.34 | 1.55 | 4.40 | 7 out of 8 |
| | Commercial | 4C | 10x LoD | 34.09 | 0.82 | 2.39 | 7 out of 8 |
| | Commercial | 15-30C | 10x LoD | 34.12 | 0.86 | 2.53 | 7 out of 8 |
| | Commercial | 4C | 3x LoD | 35.31 | 1.34 | 3.78 | 6 out of 8 |
| | Commercial | 15-30C | 3x LoD | 35.71 | 0.82 | 2.28 | 6 out of 8 |
| **Day 3** | Stabilizer Solution | 4C | 10x LoD | 33.76 | 0.55 | 1.63 | 8 out of 8 |
| | Stabilizer Solution | 15-30C | 10x LoD | 33.49 | 0.24 | 0.71 | 8 out of 8 |
| | Stabilizer Solution | 4C | 3x LoD | 34.58 | 0.75 | 2.17 | 6 out of 8 |
| | Stabilizer Solution | 15-30C | 3x LoD | 35.15 | 0.98 | 2.80 | 5 out of 8 |
| | Commercial | 4C | 10x LoD | 34.17 | 0.97 | 2.83 | 5 out of 8 |
| | Commercial | 15-30C | 10x LoD | 33.19 | 0.51 | 1.52 | 8 out of 8 |
| | Commercial | 4C | 3x LoD | 35.57 | 1.06 | 2.99 | 7 out of 8 |
| | Commercial | 15-30C | 3x LoD | 35.45 | 0.58 | 1.63 | 5 out of 8 |
| **Day 7** | Stabilizer Solution | 4C | 10x LoD | 33.35 | 0.64 | 1.92 | 8 out of 8 |
| | Stabilizer Solution | 15-30C | 10x LoD | 33.42 | 1.82 | 5.45 | 8 out of 8 |
| | Stabilizer Solution | 4C | 3x LoD | 35.26 | 1.29 | 3.67 | 8 out of 8 |
| | Stabilizer Solution | 15-30C | 3x LoD | 34.15 | 0.59 | 1.74 | 8 out of 8 |
| | Commercial | 4C | 10x LoD | 33.90 | 0.46 | 1.35 | 7 out of 8 |
| | Commercial | 15-30C | 10x LoD | 33.36 | 0.91 | 2.71 | 7 out of 8 |
| | Commercial | 4C | 3x LoD | 35.63 | 0.91 | 2.56 | 5 out of 8 |
| | Commercial | 15-30C | 3x LoD | 35.42 | 1.22 | 3.43 | 6 out of 8 |
| **Day 14** | Stabilizer Solution | 4C | 10x LoD | 32.91 | 0.43 | 1.30 | 8 out of 8 |
| | Stabilizer Solution | 15-30C | 10x LoD | 33.24 | 0.31 | 0.92 | 8 out of 8 |
| | Stabilizer Solution | 4C | 3x LoD | 34.42 | 0.68 | 1.98 | 8 out of 8 |
| | Stabilizer Solution | 15-30C | 3x LoD | 34.24 | 0.80 | 2.32 | 8 out of 8 |
| | Commercial | 4C | 10x LoD | 35.94 | 0.58 | 1.60 | 3 out of 8 |
| | Commercial | 15-30C | 10x LoD | 33.86 | 0.56 | 1.66 | 8 out of 8 |
| | Commercial | 4C | 3x LoD | 36.82 | | | 1 out of 7 |
| | Commercial | 15-30C | 3x LoD | 35.58 | 0.67 | 1.87 | 8 out of 8 |
| **Day 21** | Stabilizer Solution | 4C | 10x LoD | 32.51 | 0.37 | 1.14 | 8 out of 8 |
| | Stabilizer Solution | 15-30C | 10x LoD | 33.26 | 0.28 | 0.85 | 8 out of 8 |
| | Stabilizer Solution | 4C | 3x LoD | 34.15 | 0.36 | 1.04 | 8 out of 8 |
| | Stabilizer Solution | 15-30C | 3x LoD | 35.35 | 2.02 | 5.70 | 7 out of 8 |
| | Commercial | 4C | 10x LoD | 35.85 | 0.31 | 0.87 | 2 out of 8 |
| | Commercial | 15-30C | 10x LoD | 34.46 | 0.69 | 2.00 | 8 out of 8 |
| | Commercial | 4C | 3x LoD | No Ct | No Ct | No Ct | 0 out of 8 |
| | Commercial | 15-30C | 3x LoD | 36.07 | 0.69 | 1.92 | 8 out of 8 |
| **Day 28** | Stabilizer Solution | 4C | 10x LoD | 32.91 | 0.50 | 1.53 | 8 out of 8 |
| | Stabilizer Solution | 15-30C | 10x LOD | 33.16 | 0.59 | 1.78 | 8 out of 8 |
| | Stabilizer Solution | 4C | 3x LoD | 34.98 | 0.89 | 2.54 | 8 out of 8 |
| | Stabilizer Solution | 15-30C | 3x LoD | 35.57 | 1.58 | 4.43 | 5 out of 8 |
| | Commercial | 15-30C | 10x LoD | 35.13 | 0.81 | 2.30 | 5 out of 8 |
| | Commercial | 15-30C | 3x LoD | 36.47 | 1.29 | 3.53 | 5 out of 8 |

## Claims

1. A method of stabilizing a urine sample from a subject for nucleic acid analysis, the method comprising adding an aliquot of the sample to a stabilizer solution consisting of 1-5.5 M guanidine thiocyanate, 2-10% v/v Triton-X-100, and 20-100 mM sodium acetate at pH 4.1 to form a stabilized sample.

2. The method of claim 1, wherein the ratio of stabilizer solution volume to sample aliquot volume is in the range from 0.04 to 0.25.

3. The method of claim 2, wherein the ratio is 0.2.

4. The method of claim 3, wherein 0.4 ml of stabilizer solution is added to 2 ml of urine.

5. The method of claim 1 further comprising adding a known amount of one or more reference nucleic acid sequences to the aliquot of sample or to the stabilized sample.

6. The method of claim 1 further comprising adding silica coated magnetic beads to the aliquot of sample or to the stabilized sample.

7. The method of claim 1, wherein the stabilized sample is subjected to a nucleic acid amplification reaction.

8. The method of claim 1, wherein the sample is from a subject suspected of having an infection.

9. The method of claim 8, wherein the infection is caused at least in part by a pathogen selected from the group consisting of *Chlamydia trachomatis* and *Neisseria gonorrhoeae.*

10. Use of a stabilizer solution as defined in claim 1, in a method of screening for the presence of a pathogen in a urine sample by nucleic acid analysis, the method comprising:
(a) adding the stabilizer solution of claim 1 to an aliquot of the urine sample to form a stabilized urine sample;
(b) storing the stabilized sample at 15-30 °C.;
(c) adding magnetic particles to the stabilized sample, wherein the particles bind nucleic acids from the stabilized sample;
(d) isolating the bound nucleic acids from step (c) using a magnet;
(e) amplifying one or more target nucleic acids from the isolated nucleic acids obtained in step (d) using one or more primer sets specific for the pathogen, wherein the step of amplifying comprises performing quantitative polymerase chain reaction and the presence or absence of the pathogen in the sample is indicated by the Ct value from the reaction.

11. The use of claim 10, wherein the pathogen is selected from the group consisting of *Chlamydia trachomatis* and *Neisseria gonorrhoeae.*

## Patentansprüche

1. Verfahren zur Stabilisierung einer Urinprobe von einem Patienten für die Nukleinsäureanalyse, wobei man bei dem Verfahren eine Teilmenge der Probe zu einer aus 1-5,5 M Guanidinthiocyanat, 2-10 Vol.-% Triton-X-100 und 20-100 mM Natriumacetat bei pH 4,1 bestehenden Stabilisatorlösung gibt, so dass eine stabilisierte Probe gebildet wird.

2. Verfahren nach Anspruch 1, wobei das Verhältnis von Stabilisatorlösungsvolumen zu Probenteilmengenvolumen im Bereich von 0,04 bis 0,25 liegt.

3. Verfahren nach Anspruch 2, wobei das Verhältnis 0,2 beträgt.

4. Verfahren nach Anspruch 3, wobei 0,4 ml Stabilisatorlösung zu 2 ml Urin gegeben werden.

5. Verfahren nach Anspruch 1, bei dem man ferner eine bekannte Menge einer oder mehrerer Referenznukleinsäuresequenzen zur Probenteilmenge oder zur stabilisierten Probe gibt.

6. Verfahren nach Anspruch 1, bei dem man ferner mit Siliciumdioxid beschichtete Magnetkügelchen zur Probenteilmenge oder zur stabilisierten Probe gibt.

7. Verfahren nach Anspruch 1, wobei die stabilisierte Probe einer Nukleinsäureamplifikationsreaktion unterzogen wird.

8. Verfahren nach Anspruch 1, wobei die Probe aus einem Patienten stammt, bei dem eine Infektion vermutet wird.

9. Verfahren nach Anspruch 8, wobei die Infektion wenigstens zum Teil durch einen Krankheitserreger verursacht wird, der aus der aus *Chlamydia trachomatis* und *Neisseria gonorrhoeae* bestehenden Gruppe ausgewählt ist.

10. Verwendung einer Stabilisatorlösung mit der in Anspruch 1 angegebenen Bedeutung bei einem Verfahren zum Screening auf das Vorliegen eines Krankheitserregers in einer Urinprobe mittels Nukleinsäureanalyse, wobei man bei dem Verfahren:
(a) die Stabilisatorlösung aus Anspruch 1 zu einer Teilmenge der Urinprobe gibt, so dass eine stabilisierte Urinprobe gebildet wird;
(b) die stabilisierte Probe bei 15-30 °C lagert;
(c) Magnetpartikel zur stabilisierten Probe gibt, wobei die Partikel Nukleinsäuren aus der stabilisierten Probe binden;
(d) die gebundenen Nukleinsäuren aus Schritt (c) unter Verwendung eines Magneten isoliert;
(e) eine oder mehrere Zielnukleinsäuren aus den in Schritt (d) erhaltenen isolierten Nukleinsäuren unter Verwendung eines oder mehrerer für den Krankheitserreger spezifischen Primersätze amplifiziert, wobei der Amplifizierungsschritt das Durchführen einer quantitativen Polymerasekettenreaktion umfasst und das Vorliegen oder Nichtvorliegen des Krankheitserregers in der Probe durch den Ct-Wert aus der Reaktion angezeigt wird.

11. Verwendung nach Anspruch 10, wobei der Krankheitserreger aus der aus *Chlamydia trachomatis* und *Neisseria gonorrhoeae* bestehenden Gruppe ausgewählt ist.

## Revendications

1. Procédé de stabilisation d'un échantillon d'urine provenant d'un sujet en vue d'une analyse d'acide nucléique, le procédé comprenant l'addition d'un aliquote de l'échantillon à une solution de stabilisant consistant en 1 à 5,5 M de thiocyanate de guanidine, 2 à 10 % v/v de triton-X-100, et 20 à 100 mM d'acétate de sodium au pH de 4,1 pour former un échantillon stabilisé.

2. Procédé suivant la revendication 1, dans lequel le rapport du volume de la solution de stabilisant au volume de l'aliquote d'échantillon va de 0,04 à 0,25.

3. Procédé suivant la revendication 2, dans lequel le rapport est de 0,2.

4. Procédé suivant la revendication 3, dans lequel on ajoute 0,4 ml de solution de stabilisant à 2 ml d'urine.

5. Procédé suivant la revendication 1, dans lequel en outre on ajoute une quantité connue d'une ou de plusieurs séquences témoins d'acide nucléique à l'aliquote de l'échantillon ou à l'échantillon stabilisé.

6. Procédé suivant la revendication 1, dans lequel on ajoute, en outre, des perles magnétiques revêtues de silice à l'aliquote de l'échantillon ou à l'échantillon stabilisé.

7. Procédé suivant la revendication 1, dans lequel on soumet l'échantillon stabilisé à une réaction d'amplification d'acide nucléique.

8. Procédé suivant la revendication 1, dans lequel l'échantillon provient d'un sujet dont on soupçonne qu'il a une infection.

9. Procédé suivant la revendication 8, dans lequel l'infection est provoquée au moins en partie par un pathogène choisi dans le groupe consistant en *Chlamydia trachomatis* et *Neisseria gonorrhoeae.*

10. Utilisation d'une solution de stabilisant telle que définie à la revendication 1, dans un procédé de criblage de la présence d'un pathogène dans un échantillon d'urine par une analyse d'acide nucléique, procédé dans lequel :
(a) on ajoute la solution de stabilisant de la revendication 1 à un aliquote de l'échantillon d'urine pour former un échantillon d'urine stabilisé,
(b) on garde l'échantillon stabilisé entre -15 et 30°C,
(c) on ajoute des particules magnétiques à l'échantillon stabilisé, les particules fixant des acides nucléique de l'échantillon stabilisé,
(d) on isole les acides nucléique fixées au stade (c) en utilisant un aimant,
(e) on amplifie un ou plusieurs acides nucléique cibles parmi les acides nucléique isolés obtenus au stade (d) en utilisant un ou plusieurs jeux d'amorces spécifiques au pathogène, le stade d'amplification comprenant effectuer une réaction quantitative en chaîne de la polymérase et la présence ou l'absence du pathogène dans l'échantillon est indiquée par la valeur Ct de la réaction.

11. Utilisation suivant la revendication 10, dans lequel le pathogène est choisi dans le groupe consistant en *Chlamydia trachomatis* et *Neisseria gonorrhoeae.*
